# EUROPEAN PATENT APPLICATION

(11) **EP 4 837 958 A1**
(43) Date of publication of application: **11.11.2026**
(21) Application number: 24923593.8
(22) Date of filing: 30.12.2024
(51) Int. Cl.: C12N 1/20, A61K 8/99, A23C 9/152, A23C 9/123, C12P 7/56, A61K 35/745, A61K 35/747

(54) **BIFIDOBACTERIUM LONGUM SUBSP. INFANTIS YLGB-1496 BACTERIAL PREPARATION, PREPARATION METHOD, AND USE THEREOF FOR INTESTINAL HEALTH**

(30) Priority: 07.02.2024 CN 202410174852
(71) Applicant: Inner Mongolia Yili Industrial Group Co., Ltd., Huhhot City, Inner Mongolia 010000 (CN)
(72) Inventor: LAN, Hanglian, Huhhot, Inner Mongolia 010000 (CN); HONG, Weilian, Huhhot, Inner Mongolia 010000 (CN); HE, Jian, Huhhot, Inner Mongolia 010000 (CN); JIANG, Qiuyue, Huhhot, Inner Mongolia 010000 (CN); ZENG, Zhaozhong, Huhhot, Inner Mongolia 010000 (CN); FANG, Bing, Huhhot, Inner Mongolia 010000 (CN); WANG, Ran, Huhhot, Inner Mongolia 010000 (CN)
(74) Representative: Gao, Lin
(86) International application number: PCT/CN2024/144038
(87) International publication number: WO 2025/167391

(57) **Abstract**

A Bifidobacterium longum subsp. infantis YLGB-1496 bacterial preparation, a preparation method, and a use thereof. The bacterial preparation is prepared by fermentation of a viable bacterium of Bifidobacterium longum subsp. infantis, and is prepared from the viable bacterium of Bifidobacterium longum subsp. infantis and its metabolite of specific composition; and the bacterial preparation is used for intestinal health regulation, has efficacies such as an anti-inflammatory efficacy, enhanced gut immunity, regulation of gut microbiota. and alleviation of intestinal bloating, can be used for the preparation of fermented foods, drugs, or animal feed, and has wide application prospects.

## Description

### CROSS REFERENCE TO THE RELATED APPLICATIONS

The present invention claims priority to Chinese Patent Application No. 2024101748526, filed with the China National Intellectual Property Administration on February 7, 2024, and entitled "BIFIDOBACTERIUM LONGUM SUBSP. INFANTIS YLGB-1496 BACTERIAL PREPARATION, PREPARATION METHOD, AND USE THEREOF FOR INTESTINAL HEALTH", which is incorporated herein by reference in entirety.

### TECHNICAL FIELD

The present invention relates to the technical field of probiotics, and specifically relates to a *Bifidobacterium longum* subsp. *infantis* YLGB-1496 bacterial preparation, a preparation method therefor, and use thereof for intestinal health.

### BACKGROUND ART

Colitis (also known as nonspecific ulcerative colitis) is an inflammatory disease of the colon caused by various factors, including infections by bacteria, fungi, viruses, protozoa, parasites, etc., genetics, immune, antibiotic use, radiotherapy and other factors. Currently, there is no unified diagnostic standard for colitis. Symptoms of colitis vary depending on the specific causes of the colitis, disease duration, severity, and other factors. Common symptoms of colitis can include: mild to severe abdominal pain, persistent diarrhea, bloody purulent stool, fecal incontinence, loss of appetite, and unexplained weight loss; and severe symptoms thereof include: shortness of breath, rapid or irregular heartbeat, fever, etc.

Currently, probiotics are widely used in clinical treatments of colitis, including infantile diarrhea. However, as different probiotic brands include different bacterial strains and bacterial counts, their clinical efficacy also varies. Improving prevention and treatment of enterocolitis is of particular importance.

In view of this, the present invention is proposed.

### SUMMARY

An objective of the present invention is to provide a class of steroid synthase inhibitors and therapeutic use thereof to address the above technical problems.

The present invention is realized as follows.

In a first aspect, the present invention provides a *Bifidobacterium longum* subsp. *infantis* bacterial preparation, wherein the *Bifidobacterium longum* subsp. *infantis* bacterial preparation is prepared from viable bacteria of *Bifidobacterium longum* subsp. *infantis* and metabolites thereof, a deposit number of the *Bifidobacterium longum* subsp. *infantis* is CCTCC NO: M2011122, and the metabolites include: more than or equal to 0.156 mg/100 g of Glu-Leu, more than or equal to 0.142 mg/100 g of tanacetin, more than or equal to 0.161 mg/100 g of agavoside A, more than or equal to 0.155 mg/100 g of permetin A, more than or equal to 0.145 mg/100 g of cyclolinopeptide A, and more than or equal to 0.145 mg/100 g of desglucocoroloside.

The above metabolites are characteristic metabolites of the *Bifidobacterium longum* subsp. *infantis* bacterial preparation identified in the present invention; and levels of the above metabolites are significantly higher than those of other metabolites. Furthermore, in the *Bifidobacterium longum* subsp. *infantis*, the above metabolites exhibit stable and reproducible peaks, and possess defined molecular formulas and structures, facilitating subsequent detection and quantification.

The *Bifidobacterium longum* subsp. *infantis* bacterial preparation including the above characteristic metabolites has at least one of the following biological functions: an anti-inflammatory efficacy, effectively enhancing gut immunity, regulating gut microbiota, and alleviating intestinal flatulence or diarrhea. In addition, the probiotic postbiotic product further has excellent antioxidant effects, and strong DPPH radical scavenging capability and hydroxyl radical scavenging capability, thereby helping to enhance gut barrier function.

In a second aspect, the present invention further provides a preparation method for the *Bifidobacterium longum* subsp. *infantis* bacterial preparation, wherein the *Bifidobacterium longum* subsp. *infantis* is inoculated and subjected to fermentation, and a condition of the fermentation is a temperature of 37 ± 0.5 °C and a pH of 5.75 ± 0.5.

In a third aspect, the present invention further provides use of the *Bifidobacterium longum* subsp. *infantis* bacterial preparation, or the *Bifidobacterium longum* subsp. *infantis* bacterial preparation prepared by the above preparation method, in preparation of a nutritional additive or a fermented food, wherein the fermented food is a solid food, a liquid food, or a semi-solid food.

In a fourth aspect, the present invention further provides use of the *Bifidobacterium longum* subsp. *infantis* bacterial preparation, or the *Bifidobacterium longum* subsp. *infantis* bacterial preparation prepared by the above preparation method, in preparation of a drug or an animal food for any one of the following uses:
(1) enhancing gut immunity;
(2) regulating gut microbiota;
(3) alleviating intestinal flatulence;
(4) preventing and/or treating an inflammatory disease; and
(5) anti-aging.

Experiments have showed that the *Bifidobacterium longum* subsp. *infantis* bacterial preparation provided by the present invention has effects of enhancing gut immunity, regulating gut microbiota, alleviating intestinal flatulence, and preventing and/or treating an inflammatory disease. Therefore, the *Bifidobacterium longum* subsp. *infantis* bacterial preparation can be used for the preparation of drugs or feeds, and has wide application prospects.

In a fifth aspect, the present invention provides use of the *Bifidobacterium longum* subsp. *infantis* bacterial preparation, or the *Bifidobacterium longum* subsp. *infantis* bacterial preparation prepared by the above preparation method, in any one of the following uses:
(1) enhancing gut immunity;
(2) regulating gut microbiota;
(3) alleviating intestinal flatulence;
(4) preventing and/or treating an inflammatory disease; and
(5) anti-aging.

The present invention offers the following beneficial effects.

The viable *Bifidobacterium longum* subsp. *infantis* bacterial preparation provided by the present invention has efficacies such as an anti-inflammatory efficacy, effectively enhancing gut immunity, regulating gut microbiota and alleviating intestinal flatulence, can be used for the preparation of fermented foods, drugs, or feeds, and has wide application prospects.

### BRIEF DESCRIPTION OF DRAWINGS

To illustrate the technical solution of embodiments of the present invention clearer, drawings need to be used in embodiments will be briefly introduced below. It should be understood that the following drawings only show some embodiments of the present invention, and therefore should not be regarded as a limitation of the scope. For those ordinarily skilled in the art, other related drawings can be obtained based on these drawings without making creative efforts.
FIG. 1 shows a statistical result of a DPPH radical scavenging capacity of YLGB-1496 inactivated at different temperatures;
FIG. 2 shows a statistical result of a hydroxyl radical scavenging capacity of YLGB-1496 inactivated at different temperatures;
FIG. 3 is a statistical table of characteristic metabolite compositions of YLGB-1496 inactivated at different temperatures;
FIG. 4 is an experimental image of intestinal dissection;
FIG. 5 is a result image of H&E pathological staining of mouse ileum;
FIG. 6 is a statistical result graph of NEC scores;
FIG. 7 is a statistical result graph of survival rate in NEC;
FIG. 8 is a statistical table of expression levels of intestinal inflammatory factors;
FIG. 9 shows effects of GB1496A and GB1496D groups on the gut microbiota composition of mice at the species level (A) and the gut microbiota composition at the genus level shown as a PCoA plot (B);
FIG. 10 is difference analysis on genus level of gut microbiota among the GB1496A group, GB1496D group, and NEC group; and
FIG. 11 shows the gut microbiota composition at the species level (A) and predicted functional profile (B) of the gut microbiota.

### DETAILED DESCRIPTION OF EMBODIMENTS

To make objectives, technical solutions, and advantages of embodiments of the present invention clearer, the technical solutions of the embodiments of the present invention will be described clearly and completely below. For specific conditions not specified in the embodiments, conventional conditions or conditions recommended by the manufacturer shall be followed. Reagents or instruments used without specified manufacturers are all conventional products that are commercially available.

Features and performances of the present invention are further described in detail below in conjunction with embodiments.

In the first aspect, the present invention provides a *Bifidobacterium longum* subsp. *infantis* bacterial preparation, which is prepared from a viable bacterium of *Bifidobacterium longum* subsp. *infantis* and metabolites thereof. A deposit number of the *Bifidobacterium longum* subsp. *infantis* is CCTCC NO: M2011122, and the metabolites include: more than or equal to 0.156 mg/100 g of Glu-Leu, more than or equal to 0.142 mg/100 g of tanacetin, more than or equal to 0.161 mg/100 g of agavoside A, more than or equal to 0.155 mg/100 g of permetin A, more than or equal to 0.145 mg/100 g of cyclolinopeptide A, and more than or equal to 0.145 mg/100 g of desglucocoroloside.

The above metabolites are characteristic metabolites of the *Bifidobacterium longum* subsp. *infantis* bacterial preparation identified in the present invention, and levels of the above metabolites are significantly higher than those of other metabolites. Furthermore, in the *Bifidobacterium longum* subsp. *infantis*, the above metabolites exhibit stable and reproducible peaks, and possess defined molecular formulas and structures, facilitating subsequent detection and quantification.

The *Bifidobacterium longum* subsp. *infantis* bacterial preparation including the above characteristic metabolites has at least one of the following biological functions: anti-inflammatory efficacy, effectively enhancing gut immunity, regulating gut microbiota, and alleviating intestinal flatulence or diarrhea. In addition, the probiotic postbiotic product has excellent antioxidant effects, and strong DPPH radical scavenging capability and hydroxyl radical scavenging capability, thereby helping to enhance gut barrier function.

*Bifidobacterium longum* subsp. *infantis* with the deposit number of CCTCC NO: M2011122 is a strain YLGB-1496, and deposit information thereof refers to strain information disclosed in patent CN201910604301.8.

In optional embodiments of the present invention, a mass ratio of Glu-Leu, tanacetin, agavoside A, permetin A, cyclolinopeptide A, and desglucocoroloside in the metabolites is 1.5-1.6: 1.41-1.43: 1.6-1.62: 1.54-1.56: 1.44-1.46: 1.45-1.46.

At the above ratio, the anti-inflammatory and antioxidant effects of the *Bifidobacterium longum* subsp. *infantis* bacterial preparation can be synergistically enhanced.

In an optional embodiment of the present invention, the bacterial preparation is in a dosage form of a liquid, solid, or semi-solid. The liquid dosage form includes, but is not limited to, solution, suspension, emulsion, and semi-emulsion. The solid dosage form includes, but is not limited to, granular, powdered, and flaky forms. The semi-solid dosage form includes, but is not limited to, ointment, suppository, and paste.

In the second aspect, the present invention provides a preparation method for the *Bifidobacterium longum* subsp. *infantis* bacterial preparation, wherein the *Bifidobacterium longum* subsp. *infantis* is inoculated and subjected to fermentation, and a condition of the fermentation is a temperature of 37 ± 0.5 °C and a pH of 5.75 ± 0.5.

In an optional embodiment of the present invention, a duration of the fermentation is 11-13 h. Under the duration of the fermentation, a *Bifidobacterium longum* subsp. *infantis* bacterial preparation with a high biological activity can be obtained.

In an optional embodiment of the present invention, a stirring speed of a fermenter is 60-80 rpm; and at the stirring speed, a *Bifidobacterium longum* subsp. *infantis* bacterial preparation with a high biological activity can be obtained.

In an optional embodiment of the present invention, the fermentation is performed at a pressure of the fermenter of 0.01-0.03 MPa. Under the above condition of the fermentation, a *Bifidobacterium longum* subsp. *infantis* bacterial preparation with a high biological activity can be obtained.

In an optional embodiment of the present invention, after 9 h of the fermentation, a natural pH-reducing fermentation process is initiated and maintained until the fermentation is completed.

In one of the embodiments, prior to inoculation into the fermenter for fermentation culture, the method further includes preparation of fermentation seeds, including inoculating the cultured seeds into the fermenter and fermenting at 37 °C for 11-13 h.

In one of the embodiments, during a preparation process of the fermentation seeds, the pressure in the fermenter is 0.01-0.03 MPa, and the stirring speed of the fermenter is 70 rpm.

In an optional embodiment of the present invention, the cultured seed is prepared through a tertiary seed propagation process.

In a third aspect, the present invention further provides use of the *Bifidobacterium longum* subsp. *infantis* bacterial preparation, or the *Bifidobacterium longum* subsp. *infantis* bacterial preparation prepared by the above preparation method, in preparation of a nutritional additive or a fermented food, wherein the fermented food is a solid food, a liquid food, or a semi-solid food.

The fermented food includes, but is not limited to, foods for specified health uses and health foods, etc.

In one of the embodiments, the fermented food is a dairy product, a soy product, or a fruit and vegetable product.

In one of the embodiments, the dairy product is any one selected from a dessert, a lactic acid bacteria beverage, cheese, and yogurt; and the fruit and vegetable product is any one selected from the cucumber, carrot, beet, celery, and cabbage products.

In one of the embodiments, the above foods include, but are not limited to, beverages. There are no restrictions on a form of the food and beverage, and they can be any form of food or beverage that is generally commercially available, such as solid, liquid, fluid food form, jelly form, flaky form, granular form, or capsule form. The health foods are selected from beverages, lozenges, solid beverages, chewable tablets, capsules, granules, and drops. The above food and beverage can be prepared by using conventional methods known to those skilled in the art. During the preparation of the above food and beverage, sugars, proteins, fats, dietary fibers, vitamins, essential trace metals (e.g., manganese sulfate, zinc sulfate, magnesium chloride, potassium carbonate) for living organisms, flavors, or other compounds can further be added, provided that growth of the *Bifidobacterium longum* is not inhibited.

The bacterial preparation provided by the present invention can be prepared into general food and beverage including dairy products and fermented milk. For example, the above bacterial preparation is mixed into the cooled milk or dairy products that have been subjected to heating, mixing, homogenization, and sterilization treatment. After fermentation and cooling, pure yogurt can be produced. The above bacterial preparation can also be used in combination with other bacterial strains and/or prebiotics.

In the fourth aspect, the present invention further provides use of the *Bifidobacterium longum* subsp. *infantis* bacterial preparation or the *Bifidobacterium longum* subsp. *infantis* bacterial preparation prepared by the above preparation method in preparation of a composition for any one of the following uses:
(1) enhancing gut immunity;
(2) regulating gut microbiota;
(3) alleviating intestinal flatulence;
(4) preventing and/or treating an inflammatory disease; and
(5) anti-aging.

The composition is any one selected from health foods, drugs, and feeds.

Experiments have showed that the *Bifidobacterium longum* subsp. *infantis* bacterial preparation provided in the present invention has effects of enhancing gut immunity, regulating gut microbiota, alleviating intestinal flatulence, and preventing and/or treating the inflammatory disease. Therefore, the *Bifidobacterium longum* subsp. *infantis* bacterial preparation can be used for the preparation of drugs or feeds, thereby having wide application prospects.

The feed can be, for example, animal feed, including but not limited to feed for ruminants, feed for poultry, feed for fish. Examples include feed for pigs, sheep, cattle, horses, and fish.

In one of the embodiments, enhancing gut immunity is achieved by enhancing gut barrier function.

In one of the embodiments, enhancing gut barrier function includes: maintaining structural integrity of ileal villi and preventing or repairing damage to the gut barrier.

The gut barrier is a crucial barrier of the gut that can prevent harmful substances, such as pathogenic microorganisms and toxins, from entering other tissues, organs, and blood of the human body. A normal gut mucosal barrier is composed of a mechanical barrier, a chemical barrier, an immunological barrier, and a biological barrier. Using the *Bifidobacterium longum* subsp. *infantis* bacterial preparation provided by the present invention to prevent or repair damage to the mechanical barrier, the chemical barrier, the immunological barrier, and/or the biological barrier falls within the scope of protection of the present invention.

In one of the embodiments, the inflammatory disease is selected from a chronic inflammatory disease.

In one of the embodiments, the chronic inflammatory disease is selected from an inflammatory bowel disease.

In one of the embodiments, when the chronic inflammatory disease is the inflammatory bowel disease, preventing and/or treating the inflammatory bowel disease includes achieving at least one of the following uses: reducing or eliminating inflammatory cell infiltration in the colon, reducing or eliminating colonic crypt abscesses or damage, and inhibiting the level of inflammation in the intestinal tissue.

In one of the embodiments, the inflammatory bowel disease is any one selected from acute colitis, ulcerative colitis, Crohn's disease, microscopic colitis, diversion colitis, Behçet's disease with immuno-oncology colitis, chemotherapy- or radiation-induced colitis, graft-versus-host disease colitis, collagenous colitis, lymphocytic colitis, necrotizing enterocolitis, and pouchitis.

In one of the embodiments, the necrotizing enterocolitis is neonatal necrotizing enterocolitis. In particular, probiotic intervention effectively enhances digestive and absorptive capacity of the gut and promotes rapid maturation of the gut mucosal barrier and an immune system, thereby improving defense capabilities of the body, which is of great significance for healthy development of infants and young children.

In one of the embodiments, treating colitis includes increasing survival rate in patients with colitis.

In one of the embodiments, preventing colitis includes reducing incidence of colitis.

In one of the embodiments, an expression level of at least one of the following inflammatory factors: IL-6, IL-1β, IL-10, TLR-4, and TNF-α, in an intestinal tissue is inhibited.

In one of the embodiments, abundance of *Escherichia coli* in the gut is inhibited, and abundance of *Lactobacillus* and *Bifidobacterium* are increased.

In one of the embodiments, the dosage form of the drug is a tablet, pill, powder, suspension, gel, emulsion, cream, granule, nanoparticle, capsule, suppository, injection, or spray.

In one of the embodiments, the above drug is a liquid pharmaceutical formulation (such as a type of injection). For example, a solution, suspension, or gel typically includes a liquid carrier, such as water and/or a pharmaceutically acceptable organic solvent. In addition, such liquid preparation can further include a pH adjuster, an emulsifier or a dispersing agent, a buffer, a preservative, a wetting agent, a gelling agent (e.g., methylcellulose), a dye, and/or a flavor, such as those defined above. The drugs can be isotonic, that is, they can have the same osmotic pressure as blood. Isotonicity of the drugs can be adjusted using sodium chloride and other pharmaceutically acceptable reagents, and these reagents can be such as glucose, maltose, boric acid, sodium tartrate, propylene glycol, and other inorganic or organic soluble substances. Viscosity of a liquid composition can be adjusted using a pharmaceutically acceptable thickening agent, such as methylcellulose. Other suitable thickening agents include, such as xanthan gum, carboxymethylcellulose, hydroxypropylcellulose, carbomers. A preferred concentration of the thickening agent depends on the reagent selected.

In an optional embodiment, the above drug is a solid pharmaceutical preparation, such as a lyophilized bacterial powder, and a granular preparation.

In an optional embodiment, the drug is formulated for oral administration, injection administration, or intragastric administration.

In a fifth aspect, the present invention further provides use of the *Bifidobacterium longum* subsp. *infantis* bacterial preparation or the *Bifidobacterium longum* subsp. *infantis* bacterial preparation prepared by the above preparation method for any one of the following uses:
(1) enhancing gut immunity;
(2) regulating gut microbiota;
(3) alleviating intestinal flatulence;
(4) preventing and/or treating an inflammatory disease; and
(5) anti-aging.

In one of the embodiments, enhancing gut immunity is achieved by enhancing gut barrier function.

In one of the embodiments, the enhancing gut barrier function includes:

maintaining structural integrity of ileal villi, and preventing or repairing damage to the gut barrier.

In one of the embodiments, the inflammatory disease is selected from a chronic inflammatory disease.

In one of the embodiments, the chronic inflammatory disease is selected from an inflammatory bowel disease.

In one of the embodiments, when the chronic inflammatory disease is the inflammatory bowel disease, preventing and/or treating the inflammatory bowel disease includes achieving at least one of the following uses: reducing or eliminating inflammatory cell infiltration in the colon, reducing or eliminating colonic crypt abscesses or damage, and inhibiting a level of inflammation in an intestinal tissue.

In one of the embodiments, the inflammatory bowel disease is any one selected from acute colitis, ulcerative colitis, Crohn's disease, microscopic colitis, diversion colitis, Behçet's disease with immuno-oncology colitis, chemotherapy- or radiation-induced colitis, graft-versus-host disease colitis, collagenous colitis, lymphocytic colitis, necrotizing enterocolitis, and pouchitis.

In one of the embodiments, the necrotizing enterocolitis is neonatal necrotizing enterocolitis.

### Example 1

The present embodiment provides a *Bifidobacterium longum* subsp. *infantis* bacterial preparation, and a preparation method thereof is as follows.

### I. Preparation of tertiary seed

### 1. Standard cryopreservation tubes

Purified strains were used for uniformly preparing and aliquoted into 1.5mL centrifuge tubes (no fewer than 50 tubes). The tubes were stored at -80 °C with a storage period not exceeding 6 months.

### 2. Activation of cryopreservation tubes

A tube of strains stored at -80 °C was taken and thawed at room temperature. 200 µL of bacterial suspension was aseptically taken and transferred into 10 mL of seed liquid culture medium, followed by static incubation at 37 °C for 11-13 h.

### 3. Primary purification

The cultured bacterial suspension was diluted and spread-plated at dilutions of 10⁻⁴, 10⁻⁵, and 10⁻⁶. Two MRS solid plates were prepared for each dilution, and the plates were incubated inverted at 37 °C for 48 h-72 h until colonies formed on the plates. Single colonies were respectively picked up with inoculation loops and placed in 5 tubes, each containing 10 mL MRS liquid medium. The selected colonies were uniform in size and were subjected to static incubation at 37 °C for 11-13 h.

### 4. Secondary purification

The cultured bacterial suspension obtained from the primary purification was diluted and spread-plated at dilutions of 10⁻⁴, 10⁻⁵, and 10⁻⁶. Two MRS solid plates were prepared for each dilution, and the plates were incubated inverted at 37 °C for 48 h-72 h until colonies formed on the plates. Single colonies were respectively picked up with inoculation loops and placed in 5 tubes, each containing 10 mL MRS liquid medium. The selected colonies were uniform in size and were subjected to static incubation at 37 °C for 11-13 h.

### 5. Preparation of primary seed

A tube of the cultured bacterial suspension from the secondary purification was selected. Then, 200 µL of the bacterial suspension was respectively pipetted with a pipette into 5 tubes, each containing 10 mL of MRS liquid medium, followed by static incubation at 37 °C for 11-13 h.

### 6. Preparation of secondary seed

Four tubes of the cultured primary seeds were selected. 4 mL was respectively pipetted into four bottles each containing 80 mL of MRS liquid medium, followed by static incubation at 37°C for 11-13 h.

### 7. Preparation of tertiary seed

The cultured secondary seed was respectively poured into two bottles, each containing 1.8 L of MRS liquid medium, followed by static incubation at 37°C for 12-14 h.

### 8. Temporary storage of tertiary seed

The tertiary seed can be stored at 4 °C for no more than 6 h after the preparation was completed.

### II. Preparation of fermented seed

### 1. Inoculation

(1) An agitator and a temperature control program were turned on, with the stirring speed of 70 rpm and the temperature of 37 °C.
(2) A nitrogen inlet valve was opened and a small flow of nitrogen was introduced into the fermenter to maintain the fermenter under a positive pressure for 5-10 min.
(3) During inoculation, alcohol was poured onto the inoculation loop and ignited to create a flame ring.
(4) A top port of the inoculator was unscrewed, and the cultured seed was poured into a sterile zone above the flame, with an inoculation volume of 2.5%.
(5) After inoculation, a top cap of the inoculator was tightly screwed, an inoculation valve was closed, and an alcohol flame ring was extinguished.
(6) The nitrogen inlet valve and exhaust valve were closed, and the pressure of the fermenter was controlled between 0.01-0.03 MPa for pressure-maintained fermentation.

### 2. Fermentation

(1) Parameters of the fermentation were set, with the stirring speed of 70 rpm and the temperature of 37 °C.
(2) A duration of the fermentation was 11 h-13 h, during which a fermentation status, including pH, OD600, temperature, and stirring speed, was monitored every 2 h.
(3) Upon completion of the fermentation, it can be inoculated into the fermenter or cooled to 10-20 °C and stored for no more than 6 h.

### III. Culture in fermenter

### 1. Inoculation

(1) The agitator and the temperature control program were turned on, with the stirring speed of 70 rpm and the temperature of 37 °C.
(2) An inoculation pipeline was subjected to steam sterilization for 30 min.
(3) The nitrogen inlet valve was opened, nitrogen was introduced for 5-10 min, then a bottom valve of the seed tank and the inoculation valve of the fermenter were opened for inoculation, with an inoculation volume of 2%.
(4) After inoculation was completed, the inoculation valve of the fermenter was closed, and the inoculation pipeline was cleaned.
(5) The nitrogen inlet valve and the exhaust valve were closed, and the pressure of the fermenter was controlled at 0.01-0.03 MPa for pressure-maintained fermentation.

### 2. Fermentation

(1) Parameters of the fermentation were set, with the stirring speed of 70 rpm, the temperature of 37 °C, and a constant pH value of 5.75 for fermentation.
(2) A duration of the fermentation was 11-13 h, during which the fermentation status, including pH, OD 600, temperature, and stirring speed, was monitored every 2 h.
(3) After 9 h of fermentation, a natural pH-reducing fermentation process was initiated, once the pH value dropped below 4.2, the fermentation was terminated, and inactivation of the bacteria can be carried out, or the culture was cooled to 10-20 °C and stored for no more than 4 h.

### 4. Lyophilization

Excipients were mixed with fermentation broth, followed by lyopilization.

Formulation of the excipient was as follows.

| Name of Raw Material | Amount (kg/100kg) |
|---|---|
| Skimmed Milk Powder | 10.0 |
| Trehalose | 5.0 |
| Glycerol | 1.0 |
| Sodium Erythorbate | 0.5 |
| Purified Water | 83.5 |

Procedures of the lyophilization were as follows.
(1) Personnel requirements in an aseptic workshop
   Operating personnel entered a production area in accordance with the "Personnel Purification Operating Procedures for the Workshop Area".
(2) Tray loading
   Pressure of an intermediate tank was adjusted to 0.10-0.12 MPa, and the bacterial suspension was forced into freeze-drying trays through the pipeline by pressure. Weight of the tray loading was: 1.6-1.8 kg per tray. The freeze-drying trays were placed into a freeze-dryer in sequence, a temperature probe was inserted, and a chamber door was closed.
(3) Freeze-drying process
   The freeze-dryer was turned on and a program was set according to the freeze-drying process.

Parameters of the freeze-drying process were as follows.

| Stage | Temperature | Duration | Vacuum Degree |
|---|---|---|---|
| Pre-freezing | -40 °C | 2 h | - |
| Sublimation Drying-Stage One | -40 °C | 1 h | 30~50 Pa |
| | -15 °C | 1 h | 30~50 Pa |
| | -15 °C | 12 h | 20~30 Pa |
| Sublimation Drying-Stage Two | 10 °C | 1 h | 20~30 Pa |
| | 10 °C | 10 h | 20~30 Pa |
| Desorption Drying | 25 °C | 1 h | 10~20 Pa |
| | 25 °C | 8 h | 10~20 Pa |
| | 25 °C | 12 h | 3~10 Pa |

### (4) Completion of freeze-drying

After the freeze-drying program was completed, a chamber air inlet valve and a condenser air inlet valve were opened. Once the pressure inside the chamber was normal atmospheric pressure, the chamber door was opened to take out materials.

### Example 2

The present example compares effects of different inactivation processes on antioxidant functions of postbiotics of the strains and hydroxyl radical scavenging capacities of YLGB-1496 inactivated at different temperatures.
I. The inactivated bacteria were prepared by the following method. Conditions of the fermentation were the same as those in Example 1, and inactivation of the bacteria was carried out after the fermentation was completed.

### 1. Inactivation of bacteria

### (1) Sterilization conditions for the strain were as follows.

The sterilization condition for the *Bifidobacterium longum* subsp. *infantis* YLGB-1496 was 90 °C for 15 min.

(2) After inactivation, the bacteria can be centrifuged or cooled to 10-20 °C and stored for no more than 4 h.

### 2. Centrifugal separation

(1) An operating water supply valve of the centrifuge was opened, a total pressure of an operating water pipeline was above 3 bar, and a mechanical seal water pressure was maintained at 1.8-2.5 bar.
(2) The centrifuge was started, when a rotational speed of the centrifuge reached 11,600-11,800 rpm, the system automatically performed a discharge operation, and a bowl indicator on a main interface remained continuously illuminated.
(3) A feed valve was opened to allow materials to enter the centrifuge bowl and begin a centrifugation operation.
(4) Parameters of the centrifugation process were set as a feed rate of 600 L/h and a duration of discharge of 150 s.
(5) Upon completion of the centrifugation, a protective agent was added based on weight of a bacterial paste.

### 3. Mixing excipient

(1) An excipient pipeline was sterilized with steam for 30 min.
(2) The excipient was added at a 1:1 ratio based on a weight of the bacterial paste.
(3) After the excipient was added, the mixture was stirred for 5-10 min to ensure uniform mixing.
(4) After the excipient was mixed, the materials were transferred to an intermediate tank and stirred for 5-10 min before proceeding to the subsequent steps of freeze-drying and tray loading.

Formulation of the excipient was as follows.

| Name of Raw Material | Amount (kg/100kg) |
|---|---|
| Skimmed Milk Powder | 10.0 |
| Trehalose | 5.0 |
| Glycerol | 1.0 |
| Sodium Erythorbate | 0.5 |
| Purified Water | 83.5 |

### 4. Freeze-drying

(1) Personnel requirements in the aseptic workshop
   Operating personnel entered the production area in accordance with the "Personnel Purification Operating Procedures for the Workshop Area."
(2) Tray loading
   Pressure of the intermediate tank was adjusted to 0.10-0.12 MPa, and the bacterial paste was forced into the freeze-drying trays through the pipeline by pressure. Weight of the tray loading was: 1.6-1.8 kg per tray. The freeze-drying trays were placed into the freeze-dryer in sequence, the temperature probe was inserted, and the chamber door was closed.
(3) Freeze-drying process
   The freeze-dryer was turned on and the program was set according to the freeze-drying process.

Parameters of the freeze-drying process were as follows.

| Stage | Temperature | Duration | Vacuum Degree |
|---|---|---|---|
| Pre-Freezing | -40 °C | 2 h | - |
| Sublimation Drying-Stage One | -40 °C | 1 h | 30~50 Pa |
| | -15 °C | 1 h | 30~50 Pa |
| | -15 °C | 12 h | 20~30 Pa |
| Sublimation Drying-Stage Two | 10 °C | 1 h | 20~30 Pa |
| | 10 °C | 10 h | 20~30 Pa |
| Desorption Drying | 25 °C | 1 h | 10~20 Pa |
| | 25 °C | 8 h | 10~20 Pa |
| | 25 °C | 12 h | 3~10 Pa |

### (4) Completion of freeze-drying

After the freeze-drying program was completed, a chamber air inlet valve and a condenser air inlet valve were opened. Once the pressure inside the chamber was normal atmospheric pressure, the chamber door was opened to take out materials.

### II. Methods for determining antioxidant activity were as follows.

### (1) 2,2-diphenyl-1-picrylhydrazyl (DPPH) radical scavenging assay

A DPPH radical scavenging assay was performed using a previous method with slight modifications. A 200 µL of 0.2 mM DPPH solution was mixed with 200 µL of bacterial suspension (10⁹ cfu/mL) and incubated at 25 °C for 30 min in the dark. An equal volume of PBS (pH 7.4) was used for a control group, and an equal amount of PBS (pH 7.4) replaced the DPPH radical solution for a blank group. After centrifugation at 2,000 × g for 10 min, absorbance of the solution at 517 nm was measured. A calculation formula was as follows: $Scavenging rate \left(%\right) = \left[1-\frac{{A}_{sample} - {A}_{blank}}{{A}_{control}}\right] \times 100 .$

### (2) Hydroxyl radical scavenging

A total of 1.0 mL of each sample was added to a mixture including 2.5 mM 1,10-phenanthroline (1.0 mL), 2.5 mM FeSO₄ (1.0 mL), and PBS (1.0 mL, pH 7.4). After adding 20 mM H₂O₂ (1.0 mL), the mixture was incubated in a water bath at 37 °C for 90 min. Absorbance was measured at 536 nm. The activity of scavenging hydroxyl radical was calculated as follows: $Scavenging rate \left(%\right) = \left(\frac{{A}_{sample} - {A}_{blank}}{{A}_{control} - {A}_{blank}}\right) \times 100 .$

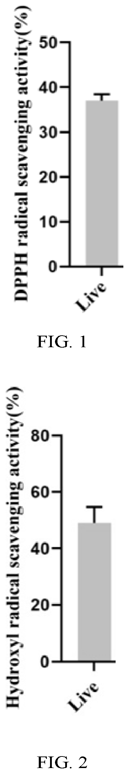

The results are as shown in FIG. 1. The DPPH scavenging capacity of viable bacteria of YLGB-1496 was 36.96%. Viable bacteria have a good DPPH radical scavenging capacity.

The results are as shown in FIG. 2. The hydroxyl radical scavenging capacity of viable bacteria of YLGB-1496 was 48.96%.

### Example 3

Characteristic metabolite composition of YLGB-1496 at different inactivation temperatures. Detection of the characteristic metabolites was performed according to the following parameters and conditions of LC-MS.

Specific method was as follows.

### 1. Sample treatment

All samples (0.5 mL) were transferred into 2 mL centrifuge tubes, and a grinding bead with a diameter of 6 mm was added, followed by adding 400 µL of extraction solution (methanol: water = 4:1 (v:v)) containing 0.02 mg/mL of internal standard (L-2-chlorophenylalanine). Subsequently, the samples were subjected to grinding in a cryogenic tissue grinder at -10 °C and 50 Hz for 6 min, followed by low-temperature ultrasonic extraction for 30 min (5 °C, 40 kHz). The samples were then allowed to stand at -20 °C for 30 min, followed by centrifuging for 15 min (13,000 g, 4 °C), and supernatant was transferred to a sample vial equipped with an inner tube for instrumental analysis. In addition, 20 µL of the supernatants were taken from individual samples, respectively, and mixed to serve as a quality control sample.

### 2. LC-MS detection

An instrument platform used for LC-MS analysis was a Thermo Fisher UHPLC-Q Exactive HF-X system (ultra-high performance liquid chromatography coupled with Fourier-transform mass spectrometry).

Chromatographic condition: the chromatographic column was an ACQUITY UPLC HSS T3 (100 mm × 2.1 mm i.d., 1.8 µm; Waters, Milford, USA); and the mobile phase A was 95% water + 5% acetonitrile (including 0.1% formic acid), and the mobile phase B was 47.5% acetonitrile + 47.5% isopropanol + 5% water (including 0.1% formic acid). An injection volume was 2 µL, and a column temperature was 40 °C.

An elution gradient of the mobile phase was as follows.

| Time (min) | Flow Rate (mL/min) | A (%) | B (%) |
|---|---|---|---|
| 0 | 0.4 | 100 | 0 |
| 3.5 | 0.4 | 75.5 | 24.5 |
| 5 | 0.4 | 35 | 65 |
| 5.5 | 0.4 | 0 | 100 |
| 7.4 | 0.6 | 0 | 100 |
| 7.6 | 0.6 | 48.5 | 51.5 |
| 7.8 | 0.5 | 100 | 0 |
| 9 | 0.4 | 100 | 0 |
| 10 | 0.4 | 100 | 0 |

Condition of mass spectrometry: the samples underwent electrospray ionization, and mass spectrometric signals were acquired using both positive and negative ion scanning modes. Specific parameters are listed in the table below.

| Description | Description | Parameter |
|---|---|---|
| Scan type | Scan type(m/z) | 70-1050 |
| Sheath gas flow rate | Sheath gas flow rate (arb) | 50 |
| Aux gas flow rate | Aux gas flow rate (arb) | 13 |
| Heater temp | Heater temp (°C) | 425 |
| Capillary temp | Capillary temp (°C) | 325 |
| Spray voltage (positive) | Spray voltage (+) (V) | 3500 |
| Spray voltage (negative) | Spray voltage(-) (V) | -3500 |
| S-Lens voltage | S-Lens RF Level | 50 |
| Collision energy | Normalized collision energy (eV) | 20, 40, 60 |
| Resolution (Full MS) | Resolution (Full MS) | 60000 |
| Resolution (MS²) | Resolution (MS²) | 7500 |

### 3. Quality control

Quality control (QC) samples were prepared by mixing equal volume of extracts from all samples. Each QC sample had the same volume as the samples and was processed and analyzed using the same method as that for analyzing samples. During a process of instrumental analysis, one QC sample was inserted every 5-15 analyzed samples to monitor stability of the entire detection process.

### 4. Data processing

Before conducting statistical analysis, a series of preprocessing procedures need to be carried out on original data. The original data were imported into a metabolomics processing software Progenesis QI (Waters Corporation, Milford, USA) for baseline filtering, peak identification, integration, retention time correction, and peak alignment. Eventually, a data matrix including retention duration, mass-to-charge ratios, and peak intensities was yielded, followed by conducting the following data preprocessing.
(1) Only the variables with non-zero values in more than 80% of any one group of samples were retained.
(2) The missing values were filled in with half of the minimum value in the original matrix.
(3) The data were normalized using total peak area normalization, and then the variables with relative standard deviation (RSD) ≥ 30% in QC samples were removed.

The raw data were imported into the metabolomics processing software Progenesis QI (Waters Corporation, Milford, USA) for library searching and identification analysis, to match MS and MSMS mass spectrometry information against metabolite databases. Primary databases utilized included HMDB (http://www.hmdb.ca/), Metlin (https://metlin.scripps.edu/), and other commercial databases, public databases, and self-constructed databases.

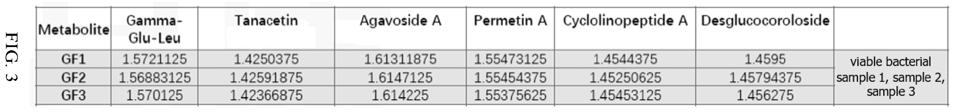

As shown in FIG. 3, the results indicate that the viable bacteria include 1.568-1.572 g/L of γGlu-Leu, 1.423-1.425 g/L of tanacetin, 1.613-1.614 g/L of agavoside A, 1.553-1.554 g/L of permetin A, 1.452-1.454 g/L of cyclolinopeptide A, and 1.456-1.459 g/L of desglucocoroloside. GF-1, GF-2 and GF-3 were viable bacterium samples 1, 2 and 3.

### Example 4

Animal experiments on NEC were conducted using inactivated GB1496 bacteria.

### 1. Animal experimental method was as follows.

Main experimental reagents were as follows.

| Reagent Name | Catalog Number | Manufacturer |
|---|---|---|
| Chloroform | 10006818 | Shanghai Reagent |
| Isopropanol | Z23-2 | Zhiyuan |
| Sterile Nuclease-Free Water | R1600-100ml | Solarbio |
| Anhydrous Ethanol | z13-3 | Tianjin Zhiyuan |
| Trizol Regant | 15596026 | invitrogen |
| 5X All-In-One RT MasterMix | G592 | abm |
| SYBR Enzyme | RR820A | TaKaRa |
| MRS Broth Medium | CM187 | Land Bridge |
| MRS Medium | CM188 | Land Bridge |
| L-Cysteine Hydrochloride | C0013-100g | Biotopped |

Preparation of solutions was as follows.
(1) Anaerobic culture medium: after boiling 1 L MRS broth medium, 0.5 g of L-cysteine hydrochloride was added and mixed well.
(2) 75% ethanol solution: 75 mL of anhydrous ethanol was mixed with 25 mL of sterile nuclease-free water.
(3) Normal saline: 8.5 g of sodium chloride was added to 1 L of deionized water.

### 1. Animal experimental method was as follows.

### A. Culture of strain YLGB1496

(1) YLGB1496 was inoculated into 10 mL of MRS broth medium including L-cysteine hydrochloride at an inoculation volume of 2%, and was activated and cultured for three generations in a constant-temperature incubator at 37 °C (using anaerobic tubes for culture).
(2) Viable bacterial suspension of YLGB1496: 10 mL of the viable bacterial suspension with MRS broth medium was centrifuged at 4,500 r for 10 min, then supernatant was discarded, and then the resultant was resuspended in sterile water. The process was repeated 2-3 times. Finally, 10 mL of sterile water was added for resuspension, from which 1 mL suspension was pipetted and transferred to a 15 mL centrifuge tube. Normal saline was then added to adjust a concentration of the bacterial suspension to 5 × 10⁷ cfu/mL, followed by storing at 4 °C for later use.
(3) Inactivated bacterial suspension of YLGB1496: the preceding steps were the same as above, and finally the bacterium suspension was placed in an 80 °C water bath for inactivation for 12 min, followed by storing at 4 °C for later use.

### B. Design of animal experiment on NEC

### (1) Grouping of experimental animals

The grouping for the animal experiment on NEC was as follows.

| Group Abbreviation | Group Name | Intragastric Condition | Number |
|---|---|---|---|
| NEC | Disease control group | Normal saline | 20 |
| Con | Normal control group | Normal saline | 18 |
| GB1496A | Viable bacterial group of GB1496 | 5×10⁷ cfu/ml of viable bacterial suspension | 17 |
| GB1496D | Inactivated bacterial group of GB1496 | 5×10⁷ cfu/ml of inactivated bacterial suspension | 17 |

(2) Husbandry of experimental animals: mice in each group were all raised normally in an SPF environment with an indoor temperature of 22-25 °C and an indoor humidity of 50%-60%. Each cage included one litter of mice including one dam and 6-8 pups, and the dam was fed with feed, while the pups were nursed by the dam. An experimental period was 4 days, calculated from the time of cage separation. Intragastric administration was performed three times daily, with an 8 h interval each time. The intragastric dosage was 50 µL per administration on the first day and 100 µL per administration on subsequent days.

(3) Mouse NEC induction method: only intragastric administration was performed on the first day. On days 2, 3, and 4, 1 hour after completion of the intragastric administration, NEC induction began. Except for mice in the Con group, mice in the other groups were placed in a homemade animal hypoxic chamber for 60 s after the intragastric administration, and the chamber was filled with 100% nitrogen to create a hypoxic environment. Subsequently, the chamber was ventilated to restore to a normoxic state, and the mice were subjected to cold stimulation by being placed in a 4 °C refrigerator for 10 min. The induction procedure was performed three times daily. A mortality was calculated 24 h after the first induction. Mice that died were immediately dissected, and if the death was determined not to be caused by the intragastric administration, it was classified as an NEC-related death.

### C. Preparation of pathological sections of mouse ileum in NEC

(1) Embedding and sectioning of mouse ileal tissue: after the mice were sacrificed, a 1 cm tissue of a terminal ileum was taken. The contents were flushed out with PBS, and the tissue was placed in a tissue embedding cassette and fixed in 4% paraformaldehyde fixative for 24 h. After fixation, the tissue was washed three times with PBS to remove excess fixative and stored in 50% ethanol. The tissue was processed in a fully automatic tissue dehydrator according to a program to remove water and finally embedded in paraffin wax for sectioning. The embedded tissue was prepared into 3.5 µm sections in a paraffin sectioning machine, and the sections were stored at room temperature.
(2) Hematoxylin and eosin (H&E) staining: the sections were placed in an oven at 65 °C for 1 h. Subsequently, the sections were treated by xylene I for 15 min, xylene II for 15 min, 100% ethanol I for 5 min, 100% ethanol II for 5 min, 95% ethanol for 5 min, 80% ethanol for 5 min, and 70% ethanol for 5 min, followed by immersion in distilled water. A deparaffinization treatment of the samples was completed. Next, the samples were stained with hematoxylin for 5 min, followed by repeated rinsing and soaking in tap water for 5 min. The samples were then stained with eosin for 30 s, rinsed with tap water, and soaked for 5 min. Dehydration and clearing were performed according to the following steps: being treated by 95% ethanol I (5 min), 95% ethanol II (5 min), 100% ethanol I (5 min), 100% ethanol II (5 min), xylene I (5 min), and xylene II (5 min). Slides were fixed and sealed with neutral resin, and observed under a microscope, and images were captured.
(3) NEC histological scoring criteria: scores were defined as follows: 0 (normal), no injury 1 (mild), slight separation of submucosa and/or lamina propria 2 (moderate), moderate separation of the submucosa and/or lamina propria and/or edema in the submucosa and muscularis 3 (severe), severe separation of the submucosa and/or lamina propria and/or severe edema in the submucosa and muscularis with focal villous sloughing 4 (necrosis), villus loss, and necrosis. When necessary, intermediate scores of 0.5, 1.5, 2.5, and 3.5 were used to more accurately assess severity of ileal injury. To determine incidence of NEC, animals with a histological score less than 2 were considered not to have developed NEC, while animals with a histological score of 2 or higher were considered to have developed NEC.

The NEC histological scoring criteria were as follows.

| Score | Phenotype |
|---|---|
| 0 | Normal |
| 1 | No injury (mild) |
| 2 | Slight separation of submucosa and/or lamina propria (moderate) |
| 3 | Moderate separation of the submucosa and/or lamina propria, and/or edema in the submucosa and muscularis (severe) |
| 4 | Severe separation of the submucosa and/or lamina propria, and/or severe edema in the submucosa and muscularis, with focal villus sloughing (necrosis), villus loss, and necrosis |

### D. Collection of blood and intestinal tissue samples, and ileal RNA extraction

(1) Entire intestinal tissue was taken from each mouse, and morphology of the intestinal tissue was photographed after straightening.
(2) The intestinal tissues from all mice were divided into three sections (small intestine, ileum, and colon), and then the ileum was further cut into three 1 cm segments; and 1 cm segment from the distal end was used for pathological sectioning, and the remaining two segments were stored at -80 °C for RT-qPCR detection. Blood was collected from all mice via decapitation, and 2-3 serum samples from each group were pooled into one sample and stored at -80 °C.
(3) Intestinal contents of all mice were collected, and 2-4 samples from each group were pooled into one sample and stored at -80 °C for gut microbiota detection.

### E. Full-length 16S rDNA sequence amplification

①Genomic DNA extraction: after the genomic DNA extraction was completed, the extracted genomic DNA was detected by 1% agarose gel electrophoresis.
② PCR amplification: specific primers with barcodes were synthesized according to designated sequencing regions. To ensure accuracy and reliability of subsequent data analysis, two conditions needed to be met: 1) amplification was performed with the lowest possible cycle number, and 2) the cycle number of amplification was kept consistent for each sample. Representative samples were randomly selected for preliminary experiments to ensure that the majority of samples can amplify products with appropriate concentrations at a minimum cycle number.
③PCR was performed using Kapa Biosystems: KAPA HiFi DNA Polymerase, and a PCR instrument used was the ABI^{GeneAmp®} 9700 model. All samples were amplified under formal experimental condition, with three replicates per sample. PCR products from the same sample were pooled and detected by 2% agarose gel electrophoresis. The PCR products were recovered by cutting the gel using the AxyPrep DNA Gel Extraction Kit (AXYGEN Company), and eluted with Tris-HCl buffer, followed by detection by 2% agarose gel electrophoresis.
④Fluorescence quantification: based on the preliminary quantification results from electrophoresis, the PCR products were quantitatively detected using a QuantiFluor^{™}-ST Blue Fluorescence Quantification System (Promega Company). After quantification, the products were mixed in appropriate proportions according to the sequencing requirements for each sample.

### PacBio SMRT third-generation sequencing

①PacBio library construction: (1) end repair: sticky ends of the fragments were first converted into blunt ends; and hairpin adapters were then ligated to both ends: a single-stranded circular adapter was ligated to each end, with the two ends of the single-stranded adapter separately ligated to a positive strand and a negative strand of the double-stranded DNA, resulting in a dumbbell-shaped ("lasso loop") structure, which is termed SMRT Bell; (2) sequences that failed to ligate to adapters were removed; and (3) single-stranded circular library was annealed with primers, and bound to polymerases that were immobilized at the bottom of zero-mode waveguides (ZMWs).
②PacBio sequencing: a SMRT Cell (single-molecule real-time reaction well) included numerous circular nano-sized wells, i.e., the above ZMWs (zero-mode waveguides), with an outer diameter over 100 nm, which was smaller than a wavelength of excitation laser (several hundred nanometers). After the laser was directed from the bottom, the laser cannot penetrate through the wells into the upper solution region, confining energy to a tiny range (a volume of 20 × 10⁻²¹ L), which was just sufficient to cover a portion to be detected. As a result, the signal originated exclusively from the small reaction region, while excess free nucleotide monomers outside the wells remained in the dark, minimizing background noise. A polymerase bound with a template DNA was immobilized at the bottom of each ZMW. Upon addition of sequencing reaction reagents, each base pairing during synthesis emitted a corresponding fluorescence signal, which was subsequently detected. Each SMRT Cell included 150,000 ZMWs, and a single-molecule DNA strand was undergoing rapid synthesis within each well. In terms of results of raw sequencing data, each base incorporated was displayed as a pulse peak. With a synthesis rate exceeding 100 bases per minute, coupled with a high-resolution optical detection system, real-time detection was achieved.

### G. Data processing and analysis

All results were expressed as mean ± variance. Student's t-test was used to assess significance of differences in microbial taxa and diversity indices. Wilcoxon rank-sum test was used to compare key taxa between groups. Differences were considered significant at P < 0.05. SPSS 26.0 operating software was used for statistical analysis, and all figures and tables were generated and processed using GraphPad.

The inactivated GB1496D bacterium referred to inactivated bacterium prepared by the method in Example 2.

2. During the development of NEC, intestinal hypoxia-ischemia leads to changes in intestinal histology, typically including intestinal flatulence and edema, and in severe cases, it causes hemorrhagic necrosis of the gut.

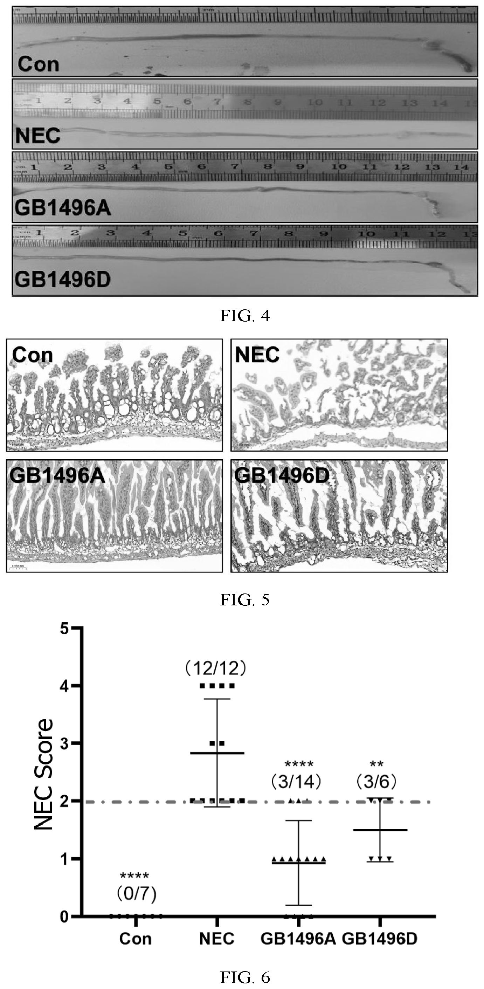

Intestinal dissection was performed on the animals, and results are shown in FIG. 4. The results indicate:
compared to the Con group, the NEC group exhibits a phenomenon of flatulence, which is one of pathological characteristics of NEC, indicating partial necrosis of cells in the gut barrier. Compared to the NEC group, the viable bacterial group of GB1496 has no phenomenon of flatulence, and the intestinal morphology appears relatively normal. Compared to the NEC group, the inactivated bacterial group of GB1496 still exhibits some degree of intestinal flatulence, but there was a certain alleviating effect.

3. Pathogenic site of NEC occurs in the terminal ileum and colon. After the onset of NEC, damage and disruption of the ileal villi are observed. Therefore, the severity of NEC is often assessed based on results of hematoxylin and eosin (H&E) staining of the ileum. H&E pathological staining experiment was performed on the ileum of the mice.

The results in FIG. 5 show that the Con group exhibits intact villus morphology and clear crypt structures; the NEC group shows severe villus damage, impaired crypt structures, and separation of a lamina propria from an epithelial layer; both the GB1496A group and the GB1496D group preserve integrity of the intestinal villi, similar to the Con group; and a comparison between the GB1496A group and the GB1496D group reveals that the integrity of the intestinal villi in the former is superior to that in the latter.

The results demonstrate that both the GB1496A group and the GB1496D group can all effectively protect structural integrity of ileal villi and prevent damage to the gut barrier, and the GB1496A group shows a superior effect compared to the GB1496D group.

4. The incidence of NEC was primarily evaluated using an NEC score, and the NEC score is based on results of H&E-staining of tissue sections of the terminal ileum. When the score is greater than or equal to 2, NEC can be diagnosed. The following is an NEC score for the incidence results of NEC.

The results are shown as in FIG. 6.

Compared to the Con group, the incidence of NEC in the NEC group increases significantly to 100% (P<0.05), indicating successful establishment of the NEC induction. The incidence in the GB1496A group (21.43%) and the GB1496D group (50%) is significantly lower than that in the NEC group (P<0.05).

### 5. Experiment of survival rate in NEC.

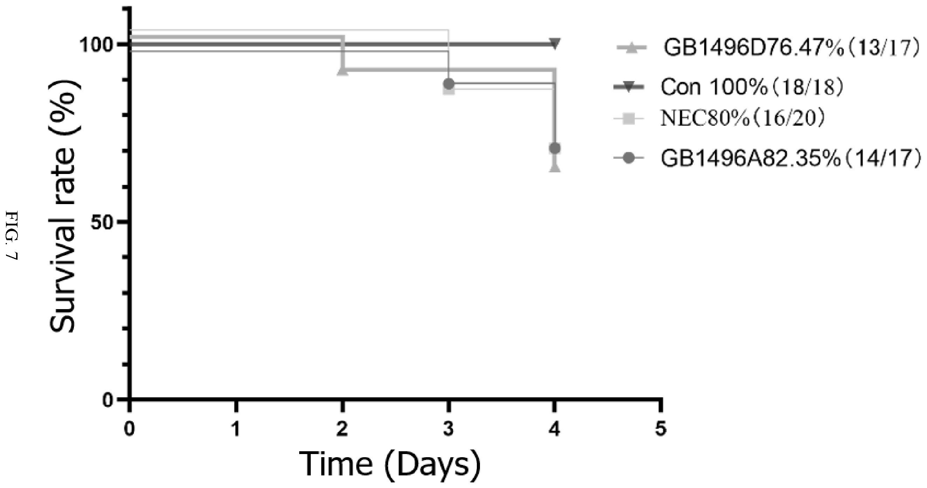

Death occurs due to diseases during the NEC induction process. As shown in FIG. 7, the NEC group, the GB1496A group, and the GB1496D group all exhibit certain mortality, but there are no statistically significant differences in survival rate among the groups (P>0.05).

Overall, a protective tend on the mice with NEC is observed in the GB1496A group and the GB1496D group. However, due to limited sample size, no significant difference is observed in this result. (Log-rank (Mantel-Cox) test, P=0.2350).

### 6. Experiment on expression levels of intestinal inflammatory factors

To investigate effects of GB1496A group and GB1496D group on intestinal inflammatory cytokines in the mice with NEC, qPCR was used to measure mRNA expression levels of these molecules in intestinal tissues. qPCR was employed to detect levels of IL-6, IL-1β, IL-10, TLR-4, and TNF-α molecules in the intestinal tissues. The primers and methods used for detection are as follows.
①RNA extraction: RNA was extracted using a Trizol method. A 50 mg sample of frozen ileal tissue was added to 1 mL of Trizol (for cell lysis) in an RNase-free homogenization tube containing glass beads. Homogenization was performed using a low-temperature homogenizer at 4°C and 8000 rpm, with each cycle lasting 30 s, and the homogenization was repeated for a total of 3 cycles. After homogenization, the samples were allowed to stand at 4 °C until foam dissipated, and the supernatant was then transferred to a 1.5 mL centrifuge tube and centrifuged at 12,000 g and 4 °C for 5 min, after which the supernatant was collected. 200 µL of chloroform was added, and the mixture was shaken vigorously for 15 s and allowed to stand at room temperature for 5 min. The resultant was centrifuged at 12,000 g and 4°C for 10 min. The supernatant was collected, 0.5 times volume of anhydrous ethanol in proportion was added and the mixture was mixed thoroughly, then transferred to a spin column, and centrifuged at 12,000 g and 4 °C for 2 min. RNA was bound to the spin column, and 0.5 mL of protein removal solution was added, followed by centrifugation at 12,000 g and 4 °C for 30 s. 0.5 mL of wash buffer was added, the resultant was allowed to stand for 2 min, and centrifuged at 12,000 g and 4 °C for 30 s, and the washing step was repeated once. 50 µL of ultrapure water was added to the center of the spin column, the resultant was allowed to stand for 2 min and then centrifuged at 12,000 g and 4 °C for 2 min. The RNA was collected in the eluate, kept on ice, and RNA concentration was measured using NanoDrop.
② Reverse transcription: a total volume was 20 µL. 4 µL of reverse transcriptase mix solution was added. The RNA was diluted with nuclease-free sterile water to achieve a concentration of 2 µg of RNA in the total reaction system, and the total reaction system was adjusted to 20 µL using nuclease-free ultrapure water. A reverse transcription program was carried out as follows: 25 °C for 10 min, 42 °C for 50 min, 85 °C for 5 min, and cooling at 4 °C. The resulting cDNA samples were stored at -20 °C.
③Real-time fluorescence quantitative PCR (RT-qPCR): 20 µL reaction system includes: 10 µL TB Green Premix reagent, 0.4 µL forward primer, 0.4 µL reverse primer, n µL of cDNA template, and (9.2-n) µL of sterile nuclease-free water. The mixture was homogenized away from light.
④ PCR conditions were as follows: pre-denaturation at 95°C for 600 s, amplification using a three-step protocol consisting of denaturation at 95°C for 20 s, annealing at a temperature of 60°C-62°C for 20 s, and extension at 72°C for 20 s, for a total of 40 cycles, followed by melting: 95°C for 10 s, 65°C for 60 s, and 97 °C for 1 s. Primer sequences and annealing temperatures are shown in Table 1-5, and β-actin was used as an internal reference gene.

Specific primer sequences are as follows.

| Gene Name | Species | Sequence | SEQ ID NO: |
|---|---|---|---|
| TLR-4 | Mouse | F:TTTATTCAGAGCCGTTGGTG | 1 |
| | | R:CAGAGGATTGTCCTCCCATT | 2 |
| IL-1β | Mouse | EF:TGGTGTGTGACGTTCCCATT | 3 |
| | | R:CAGCACGAGGCTTTTTTGTTG | 4 |
| IL-6 | Mouse | F:CCAAGAGGTGAGTGCTTCCC | 5 |
| | | R:CTGTTGTTCAGACTCTCTCCCT | 6 |
| IL-10 | Mouse | F:GCCGTCATTTTCTGCCTCAT | 7 |
| | | R:GCTTCCCTATGGCCCTCATT | 8 |
| TNF-α | Mouse | F:CCAAAGGGATGAGAAGTTCC | 9 |
| | | R:CTCCACTTGGTGGTTTGCTA | 10 |

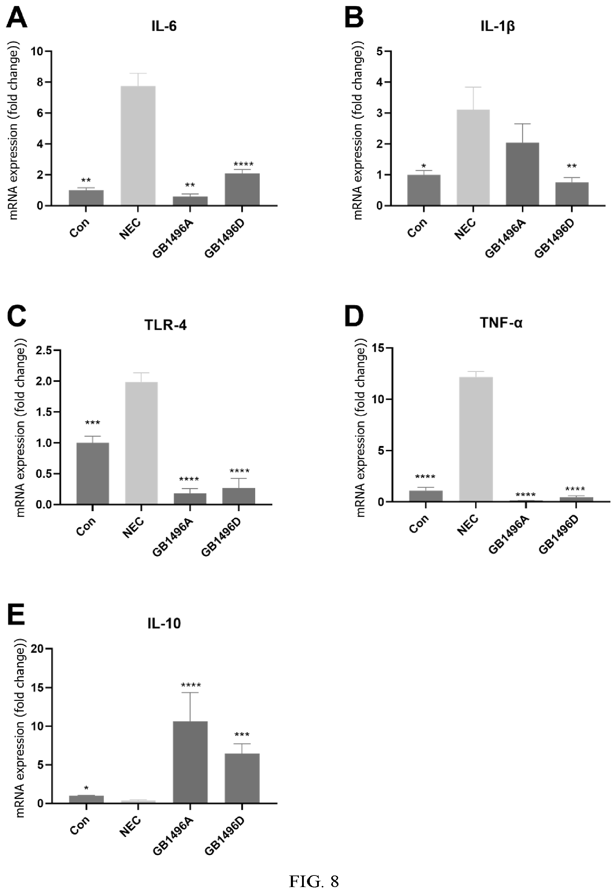

As shown in FIG. 8, compared with the Con group, the NEC group exhibits significantly increased expression levels of IL-6 (FIG. 8(A)), IL-1β (FIG. 8(B)), TNF-α (FIG. 8(D)), and TLR-4 (FIG. 8(C)), and a significant decrease in IL-10 (FIG. 8(E)). Compared with the NEC group, both GB1496A group and GB1496D group show significantly reduced expression levels of IL-6 (FIG. 8(A)), TNF-α (FIG. 8(D)), and TLR-4 (FIG. 8(C)), increased expression levels of IL-10 (FIG. 8(E)). In addition, the GB1496D group shows a significantly reduced expression level of IL-1β (FIG. 8(B)), and the GB1496A group also shows a downward trend.

As shown in FIG. 8, based on the expression levels of the above inflammatory factors, it demonstrates that GB1496A group and GB1496D group have an inhibitory effect on inflammation levels.

7. Experiment of effects on the gut microbiota of mice in the GB1496A group and the GB1496D group.

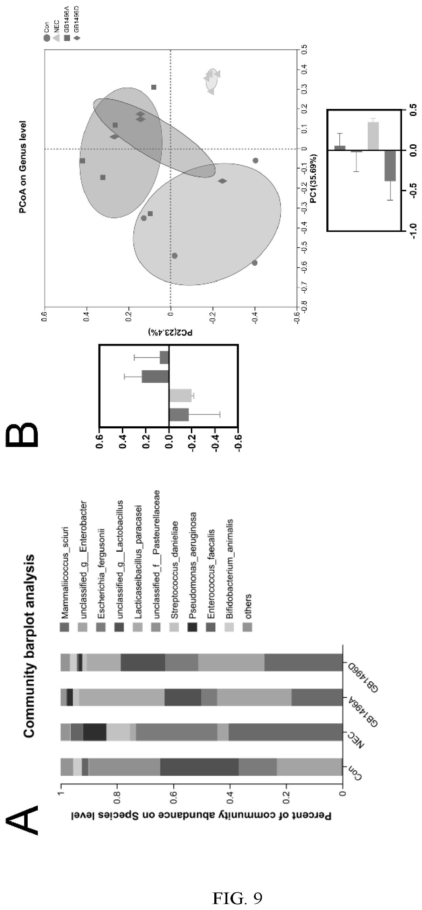

①To understand compositions of the gut microbiota of the mice in each group, 16S rDNA sequencing was employed to determine the compositions of the gut microbiota in each group. FIG. 9(A) displays the top 10 bacteria on species level for each group, namely, *Mammaliicoccus*, unclassified *Enterobacter*, *Escherichia fergusonii*, unclassified *Lactobacillus*, *Lactobacillus paracasei*, unclassified *Pasteurellaceae*, *Streptococcus danieliae*, *Pseudomonas aeruginosa*, *Enterococcus faecalis*, and *Bifidobacterium animalis*.

②Principal co-ordinates analysis (PCoA) based on Bray-Curtis distance was used to investigate similarities or differences in community composition among the samples. FIG. 9(B) compares β-diversity of the compositions of gut microbiota on genus level among the NEC group, the GB1496A group, and the GB1496D group. As shown in FIG. 9, there are significant differences in the community composition of gut microbiota between the NEC group and the other two groups, allowing for complete separation.

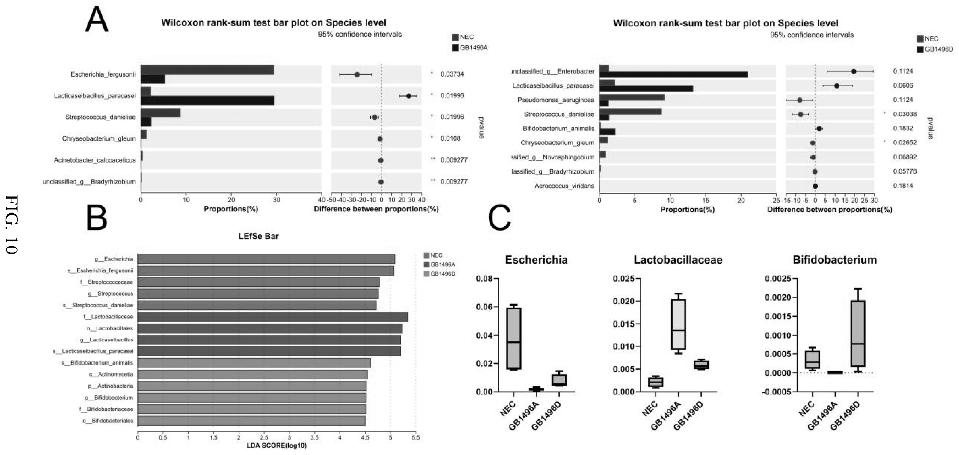

③To understand changes in the compositions of gut microbiota on genus level in NEC mice with interventions in GB1496A group and GB1496D group, the present example compares composition differences in gut microbiota on genus level among the GB1496A group, the GB1496D group and the NEC group. FIG. 10(A) shows differences in gut microbiota on genus level between the GB1496A group and the NEC group; and the abundance of pathogenic *Escherichia*, *Streptococcus*, and *Pseudomonas*, as well as the opportunistic pathogen *Acinetobacter calcoaceticus*, is significantly lower in the GB1496A group compared to the induction group, whereas the abundance of *Lactobacillus paracasei* is significantly higher in the viable bacterium group of GB1496A compared with the NEC group. FIG. 10(B) shows the differences in gut microbiota on genus level between the GB1496D group and the NEC group. Specifically, the abundance of pathogenic *Streptococcus* and *Pseudomonas* is significantly lower in the GB1496D group compared to the NEC group. The abundance of *Lactobacillus paracasei* and *Bifidobacterium* shows an increasing trend in the GB1496D group compared to the NEC group.

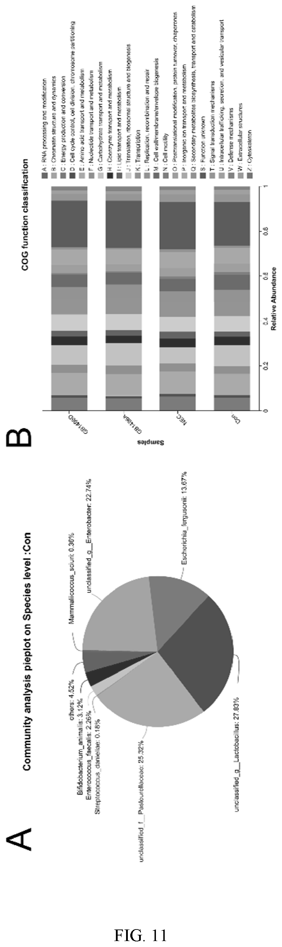

④To predict functional information regarding the microbial communities in the mouse samples, functional composition and abundance were analyzed to gain further insight into some potential microbial functional characteristics during disease progression. PICRUST2 was used to predict clusters of orthologous groups (COG) information during the course of the disease. FIG. 11 displays some functional information for each group, such as: RNA processing and modification, chromatin structure and dynamics, energy production and conversion, cell cycle control, cell division and chromosome partitioning, and amino acid transport and metabolism.

### INDUSTRIAL APPLICABILITY

The present invention provides a class of compounds that can be used as pharmaceuticals, which exhibit an inhibitory effect on steroidal synthesis enzymes with high inhibition rates. These compounds can be used for preparing steroidal synthesis enzyme inhibitors, can be used in drugs for treating hormone-dependent diseases, can be used for preparation and as therapeutic agents for hormone-dependent diseases and can be used for treating hormone-dependent diseases. The hormone-dependent diseases include at least one of the following diseases: congestive heart failure, hypertension, chronic kidney disease, diabetic nephropathy, hyperaldosteronism, cardiac fibrosis, renal syndrome, metabolic syndrome, Cushing's syndrome, insulin resistance, obesity, type 2 diabetes, breast cancer, prostate cancer, ovarian cancer, cervical cancer, diabetic foot, diabetic eye disease, diabetic ulcer, renal failure, non-alcoholic fatty liver disease, fatty liver, cirrhosis, liver fibrosis, liver cancer, pancreatic cancer, cholangiocarcinoma, colon cancer, and rectal cancer.

## Claims

1. A *Bifidobacterium longum* subsp. *infantis* bacterial preparation, **characterized in that** the *Bifidobacterium longum* subsp. *infantis* bacterial preparation is prepared from viable bacterium of *Bifidobacterium longum* subsp. *infantis* and metabolites thereof, a deposit number of the *Bifidobacterium longum* subsp. *infantis* is CCTCC NO: M2011122, and the metabolites comprise: more than or equal to 0.156 mg/100 g of Glu-Leu, more than or equal to 0.142 mg/100 g of tanacetin, more than or equal to 0.161 mg/100 g of agavoside A, more than or equal to 0.155 mg/100 g of permetin A, more than or equal to 0.145 mg/100 g of cyclolinopeptide A, and more than or equal to 0.145 mg/100 g of desglucocoroloside.

2. The *Bifidobacterium longum* subsp. *infantis* bacterial preparation according to claim 1, wherein a mass ratio of Glu-Leu, tanacetin, agavoside A, permetin A, cyclolinopeptide A, and desglucocoroloside in the metabolites is 1.5-1.6: 1.41-1.43: 1.6-1.62: 1.54-1.56: 1.44-1.46: 1.45-1.46.

3. The *Bifidobacterium longum* subsp. *infantis* bacterial preparation according to claim 1, wherein the bacterial preparation is in a form of a liquid, solid, or semi-solid.

4. A preparation method for the *Bifidobacterium longum* subsp. *infantis* bacterial preparation according to any one of claims 1 to 3, **characterized in that** the *Bifidobacterium longum* subsp. *infantis* is inoculated and subjected to fermentation, and a condition of the fermentation is a temperature of 37 ± 0.5 °C and a pH of 5.75 ± 0.5.

5. Use of the *Bifidobacterium longum* subsp. *infantis* bacterial preparation according to any one of claims 1 to 3, or the *Bifidobacterium longum* subsp. *infantis* bacterial preparation prepared by the preparation method according to claim 4, in preparation of a nutritional additive or a food, **characterized in that** the food comprises a fermented food, milk powder, liquid milk, cheese, and a snack.

6. The use according to claim 5, wherein the fermented food is a solid food, a liquid food, or a semi-solid food.

7. The use according to claim 5 or 6, wherein the fermented food is a dairy product, a soy product, or a fruit and vegetable product.

8. The use according to claim 7, wherein the dairy product is any one selected from a dessert, a lactic acid bacteria beverage, cheese, and yogurt; and the fruit and vegetable product is any one selected from cucumber, carrot, beet, celery, and cabbage products.

9. Use of the *Bifidobacterium longum* subsp. *infantis* bacterial preparation according to any one of claims 1 to 3, or the *Bifidobacterium longum* subsp. *infantis* bacterial preparation prepared by the preparation method according to any one of claims 4 to 5, in preparation of a composition for any one of following uses:
(1) enhancing gut immunity;
(2) regulating gut microbiota;
(3) alleviating intestinal flatulence;
(4) preventing and/or treating an inflammatory disease; and
(5) anti-aging.

10. The use according to claim 9, wherein the enhancing gut immunity is achieved by enhancing gut barrier function.

11. The use according to claim 9 or 10, wherein the enhancing gut barrier function comprises:
maintaining structural integrity of ileal villi, and preventing or repairing damage to the gut barrier.

12. The use according to claim 9, wherein the inflammatory disease is selected from a chronic inflammatory disease.

13. The use according to claim 9 or 12, wherein the chronic inflammatory disease is selected from an inflammatory bowel disease.

14. The use according to claim 12 or 13, wherein, when the chronic inflammatory disease is the inflammatory bowel disease, preventing and/or treating the inflammatory bowel disease comprises achieving at least one of following uses: reducing or eliminating inflammatory cell infiltration in a colon, reducing or eliminating colonic crypt abscesses or damage, and inhibiting a level of inflammation in an intestinal tissue.

15. The use according to claim 13 or 14, wherein the inflammatory bowel disease is any one selected from acute colitis, ulcerative colitis, Crohn's disease, microscopic colitis, diversion colitis, Behçet's disease with immuno-oncology colitis, chemotherapy- or radiation-induced colitis, graft-versus-host disease colitis, collagenous colitis, lymphocytic colitis, necrotizing enterocolitis, and pouchitis.

16. The use according to claim 15, wherein the necrotizing enterocolitis is neonatal necrotizing enterocolitis.

17. The use according to any one of claims 15 to 16, wherein treating colitis comprises increasing survival rate in patients with colitis.

18. The use according to any one of claims 15 to 16, wherein preventing colitis comprises reducing incidence of colitis.

19. The use according to any one of claims 15 to 16, comprising inhibiting an expression level of at least one of following inflammatory factors in the intestinal tissue: IL-6, IL-1β, IL-10, TLR-4 and TNF-α.

20. The use according to any one of claims 15 to 16, comprising inhibiting abundance of *Escherichia coli* in a gut, and increasing abundance of *Lactobacillus* and *Bifidobacterium*.

21. Use of the *Bifidobacterium longum* subsp. *infantis* bacterial preparation according to any one of claims 1 to 3 or the *Bifidobacterium longum* subsp. *infantis* bacterial preparation prepared by the preparation method according to any one of claims 4 to 5 in any one of following uses:
(1) enhancing gut immunity;
(2) regulating gut microbiota;
(3) alleviating intestinal flatulence;
(4) preventing and/or treating an inflammatory disease; and
(5) anti-aging.

22. The use according to claim 21, wherein the enhancing gut immunity is achieved by enhancing gut barrier function.

23. The use according to claim 21 or 22, wherein the enhancing gut barrier function comprises:
maintaining structural integrity of ileal villi, and preventing or repairing damage to the gut barrier.

24. The use according to claim 21, wherein the inflammatory disease is selected from a chronic inflammatory disease.

25. The use according to claim 24, wherein the chronic inflammatory disease is selected from the inflammatory bowel disease.

26. The use according to claim 24 or 25, wherein, when the chronic inflammatory disease is the inflammatory bowel disease, preventing and/or treating the inflammatory bowel disease comprises achieving at least one of following uses: reducing or eliminating inflammatory cell infiltration in a colon, reducing or eliminating colonic crypt abscesses or damage, and inhibiting a level of inflammation in the intestinal tissue.

27. The use according to claim 25 or 26, wherein the inflammatory bowel disease is any one selected from acute colitis, ulcerative colitis, Crohn's disease, microscopic colitis, diversion colitis, Behçet's disease with immuno-oncology colitis, chemotherapy- or radiation-induced colitis, graft-versus-host disease colitis, collagenous colitis, lymphocytic colitis, necrotizing enterocolitis, and pouchitis.

28. The use according to claim 27, wherein the necrotizing enterocolitis is neonatal necrotizing enterocolitis.
